# EUROPEAN PATENT APPLICATION

(11) **EP 4 417 193 A1**
(43) Date of publication of application: **21.08.2024**
(21) Application number: 21961796.6
(22) Date of filing: 28.10.2021
(51) Int. Cl.: A61K 9/127, A61K 47/46, A61P 3/10

(54) **USE OF MILK EXOSOME IN PREPARATION OF DRUG CARRIER**

(71) Applicant: Panexo Biotech Sg Pte. Ltd, Singapore 409051 (SG)
(72) Inventor: WANG, Yi, Beijing 100039 (CN); SONG, Haifeng, Beijing 100039 (CN); XU, Mingzhi, Beijing 100039 (CN); DONG, Yanan, Beijing 100039 (CN)
(74) Representative: Michalski Hüttermann & Partner Patentanwälte mbB
(86) International application number: PCT/CN2021/126995
(87) International publication number: WO 2023/070429

(57) **Abstract**

The present disclosure relates to the field of pharmaceutical preparations, and in particular to use of a milk exosome in the preparation of a drug carrier. The present disclosure uses milk exosomes as a drug carrier. The exosomes are derived from milk with safety and reliability, and has the functional properties of milk exosomes. Using the exosomes as a drug carrier can achieve sublingual administration of protein drugs. Using the milk exosomes to load liraglutide can achieve sublingual administration of liraglutide and treat type 2 diabetes.

## Description

### FIELD

The present disclosure relates to the field of pharmaceutical preparations, and in particular to use of a milk exosome in the preparation of a drug carrier and subsequent loading of active pharmaceutical ingredients.

### BACKGROUND

Exosomes refer to small membranous vesicles (30-150 nm) containing complex RNA and proteins, now specifically to spherical vesicles with a diameter of 40-100 nm. Exosomes were first discovered in sheep reticulocytes in 1983 and named "exosome" by Johnstone in 1987. A variety of cells can secrete exosomes under normal and pathological conditions. Exosomes are mainly derived from multivesicular bodies that are formed by the invagination of intracellular lysosomal particulates and released into the extracellular matrix after the outer membrane of the multivesicular bodies fuses with the cell membrane.

Currently, existing purification techniques for exosomes mainly include the following methods:

### 1. Ultracentrifugation (differential centrifugation)

Ultracentrifugation is the most commonly used means of exosome purification and is considered the gold standard for exosome extraction. However, it is limited in scale, time-consuming, and has unstable recovery rates and purities. In addition, repeated centrifugation may damage the vesicles, further impairing the quality of the final product.

### 2. Density gradient centrifugation

This is a zonal separation method, wherein the sucrose or OptiPrep solution is subjected to ultracentrifugal force to form density gradient layers that are continuously distributed from low to high. The problem existing in density gradient centrifugation is that the steps are cumbersome and time-consuming.

### 3. Ultrafiltration centrifugation

Extraction of exosomes by ultrafiltration centrifugation may cause the blockage of filter pores, resulting in a shorter membrane life and lower separation efficiency.

### 4. Immunomagnetic bead method

This method has low efficiency in extracting exosomes, and the biological activity of exosomes is easily affected by pH and salt concentration, which is not conducive to downstream experiments, making it difficult to be widely popularized.

### 5. PEG-based precipitation

Precipitation of exosomes using polyethylene glycol (PEG) has many problems such as low purity and recovery rate, many impurity proteins (false positives), non-uniform particle size, production of polymers that are difficult to remove, and destruction of exosomes by mechanical force or chemical additives such as Tween-20.

### 6. Extraction Kit

The method of extracting exosomes using commercial exosome extraction kits is small in scale and will produce a lot of impurity proteins, leading to low purity and recovery rate of exosomes.

Therefore, the present disclosure aims to provide a method for efficiently extracting milk exosomes on a large scale without relying on centrifugation.

More importantly, drug loading by exosomes is difficult, and the present disclosure achieves and improves drug loading of protein drugs by exosomes.

Currently, protein drugs are often administered via injection, which have poor patient compliance for repeated dosing and introduce safety risks.

Currently, a number of studies attempted oral delivery of drugs by using exosome as drug carrier, using the gastrointestinal tract as the primary organ of absorption. Unfortunately, such route of administration with exosome-based drug delivery system suffer from low bioavailability and does not possess any practical utility.

Therefore, the present disclosure aims to provide a formulation of drug-loaded exosomes as sublingual tablets, for highly efficient delivery and absorption of active pharmaceutical ingredient in the oral cavity.

### SUMMARY

In view of this, the present disclosure uses milk exosomes as a drug carrier. The exosomes are derived from milk with safety and reliability, and retain the normal biological properties of milk exosomes. Using the milk exosomes as a drug carrier can achieve oral administration of protein drugs. Using the milk exosomes to load liraglutide can achieve oral administration of liraglutide and treat type 2 diabetes.

In order to achieve the above objectives, the present disclosure provides the following technical solutions.

The present disclosure provides use of a milk exosome in the preparation of a drug carrier. In some specific embodiments of the present disclosure, the drug includes a protein drug. In some specific embodiments of the present disclosure, the drug includes liraglutide.

The present disclosure further provides use of a milk exosome as a drug carrier in the preparation of a medicament for the prevention and/or treatment of a disease. In some specific embodiments of the present disclosure, the drug includes a protein drug. In some specific embodiments of the present disclosure, the drug includes liraglutide. In some specific embodiments of the present disclosure, the drug is administered orally. In some specific embodiments of the present disclosure, the disease includes type 2 diabetes.

More importantly, the present disclosure further provides an oral medicament, comprising an active ingredient and a milk exosome as a drug carrier.

In the use or the oral administration of the medicament described in the present disclosure, the milk exosome is prepared by:
step 1, pre-treatment;
step 2, preliminary purification and concentration;
step 3, downstream purification;
wherein the pre-treatment comprises fat removal and/or protein removal;
the preliminarily purification and concentration are performed by tangential flow ultrafiltration, and
the downstream purification is performed via a method selected from the group consisting of CIMmultus QA column purification, Capto core 700 column purification, S400 size exclusion chromatography purification, and a combination thereof.

In some specific embodiments of the present disclosure, the fat removal in step 1 comprises:
(1) removing fat by spontaneous sedimentation and filtration; and/or
(2) removing fat by centrifugation;
wherein the filtration step comprises depth filtration and/or capsule filter filtration.

In some specific embodiments of the present disclosure, the protein removal in step 1 is carried out by a protein dissolution method and/or a protein precipitation method;
wherein the protein dissolution method includes a sodium citrate dissolution method; and
the protein precipitation method is selected from the group consisting of EDTA precipitation method, sodium phosphate precipitation method, ammonium sulfate precipitation method, and a combination thereof.

In some specific embodiments of the present disclosure, the sodium citrate dissolution method comprises the following steps: mixing an equal volume of milk and a sodium citrate solution with a mass concentration of 2%, shaking in an ice bath, and filtering through a membrane filter.

The sodium citrate dissolution method specifically comprises: pouring an equal volume of milk into a sodium citrate solution with a mass concentration of 2%, shaking in an ice bath on a shaker for 60 min to clarify the milk sample, and filtering the clarified milk sample through a 0.2 µm membrane filter.

In some specific embodiments of the present disclosure, the EDTA precipitation method comprises the following steps: mixing an equal volume of milk and an EDTA solution, standing at room temperature, centrifuging, and filtering through a membrane filter; wherein the EDTA solution has a concentration of 0.35 M and a pH of 7.0.

In some specific embodiments of the present disclosure, the sodium citrate dissolution method specifically comprises: pouring an equal volume of milk into an EDTA solution, standing at room temperature for 15 min to precipitate, centrifuging the milk sample at 12,000 rpm for 40 min after precipitation, and then filtering through 0.45 µm and 0.2 µm membrane filters.

In some specific embodiments of the present disclosure, the sodium phosphate solution has a concentration of 1 M and a pH of 6.3.

In some specific embodiments of the present disclosure, the sodium phosphate precipitation method is as follows.

Depending on the type of milk, the sodium phosphate precipitation method comprises different specific steps:
(1) Whole milk
   centrifuging the milk at 16,000 rpm for 30 min to remove fat, mixing an equal volume of milk with a sodium phosphate solution, stirring, precipitating, and freezing at -80°C;
(2) Skimmed milk
   centrifuging the milk at 16,000 rpm for 30 min to remove fat, mixing an equal volume of milk and a sodium phosphate solution, stirring, precipitating, standing at room temperature, centrifuging at 3500 rpm for 10 min, collecting the supernatant, and filtering it through a membrane filter.

In some specific embodiments of the present disclosure, the sodium phosphate precipitation method specifically comprises the following steps: centrifuging milk at 16,000 rpm for 30 min to remove fat, pouring an equal volume of milk into the sodium phosphate solution, stirring on a magnetic stirrer at 350 rpm for 15 min, and centrifuging the milk sample at 3500 rpm for 10 min after precipitation, and then sequentially filtering the supernatant through 0.8 µm, 0.45 µm and 0.2 µm membrane filters.

In some specific embodiments of the present disclosure, the ammonium sulfate precipitation method comprises the following steps: mixing an equal volume of milk and an ammonium sulfate solution, standing at room temperature, centrifuging, and filtering through a membrane filter; wherein the concentration of the ammonium sulfate solution is 3.0 M.

In some specific embodiments of the present disclosure, the ammonium sulfate precipitation method specifically comprises the following steps: pouring an equal volume of milk into an ammonium sulfate solution while gentle stirring using a glass rod, standing at room temperature for 1.5 h to precipitate, centrifuging the milk sample at 6000 rpm for 20 min after precipitation, and then sequentially filtering the supernatant through 0.8 µm, 0.45 µm and 0.2 µm membrane filter.

The present disclosure further provides a method for treating a disease, comprising administering to a subject a drug loaded in a milk exosome, wherein the milk exosome is used as a drug carrier.

In some specific embodiments of the present disclosure, the drug includes a protein drug.

In some specific embodiments of the present disclosure, for loading of a drug into milk exosomes, ultrasonication, electroporation or co-incubation approach is used.

In some specific embodiments of the present disclosure, the ultrasonication is preferably used for loading of a protein drug into exosomes. In particularly, the ultrasonication is carried out with an input power level between 450W and 600W, and a sonication time between 20min and 1h. With the help of the said ultrasonication, a better scalability and higher loading efficiency can be achieved compared to other methods.

In some specific embodiments of the present disclosure, the drug includes liraglutide, semaglutide or tirzepatide.

In some specific embodiments of the present disclosure, the drug is administered orally.

In some specific embodiments of the present disclosure, the oral administration is achieved by using sublingual tablets containing drug-loaded exosomes.

In some specific embodiments of the present disclosure, the drug-loaded exosomes are absorbed in the oral cavity via the sublingual route.

In some specific embodiments of the present disclosure, the disease includes type 2 diabetes.

The present disclosure uses milk exosomes as a drug carrier. The exosomes are derived from milk with safety and reliability, and retain the normal biological functions of milk exosomes. Using the milk exosomes as a drug carrier can achieve sublingual administration and subsequently efficient absorption of protein drugs. Using the milk exosomes to load liraglutide can achieve sublingual administration of liraglutide and treat type 2 diabetes.

### BRIEF DESCRIPTION OF DRAWINGS

For more clearly illustrating embodiments of the present disclosure or the technical solutions in the prior art, drawings for describing the embodiments or the prior art will be briefly described hereinafter.
FIG. 1 shows the process route for extraction of milk exosomes by TFF+BE-SEC and UC.
FIG. 2 shows the morphology of exosomes extracted by three methods under TEM (Bar=500 nm) (A: TFF; B: TFF + BE-SEC; C: UC).
FIG. 3 shows the particle size distribution of exosomes extracted by three methods (A: TFF; B: TFF + BE-SEC; C: UC).
FIG. 4 shows the SEC-HPLC chromatogram of milk exosomes extracted by three methods (A: TFF; B: TFF + BE-SEC; C: UC).
FIG. 5 shows a liraglutide solution after ultrasonication (left: a colloidal solution with more precipitates after ultrasonication) and a mixture of milk exosomes and liraglutide (right).
FIG. 6 shows the result obtained from Capto Core 700 column purification of milk exosomes loaded with liraglutide by ultrasonication.
FIG. 7 shows the result obtained from Capto Core 700 column purification of ultrasonicated liraglutide.
FIG. 8 shows the result obtained from QA column purification of milk exosomes and liraglutide after ultrasonication.
FIG. 9 shows the result obtained from QA column purification of ultrasonicated liraglutide.
FIG. 10 shows the effects of the liraglutide solution and the exosome-liraglutide mixture after ultrasonication and Capto Core 700 column purification on luciferase expression (all positive controls are treatment groups of 0.375 µg/ml liraglutide without ultrasonication).
FIG. 11 shows the conditions for exploring the use of electroporation to load PKH67 in exosomes; wherein, FIG. 11A shows the FITC positive rate and the particle concentration of the samples after electroporation of the PKH67-exosome mixture under different voltages (100 µF capacitance); and FIG. 11B shows the FITC positive rate and the particle concentration of the samples after electroporation of the PKH67-exosome mixture under different capacitance (500V voltage).
FIG. 12 shows the result obtained from Capto Core 700 column purification of milk exosomes loaded with liraglutide by electroporation.
FIG. 13 shows the result obtained from Capto Core 700 column purification of electroporated liraglutide.
FIG. 14 shows the effects of the liraglutide solution and the exosome-liraglutide mixture after electroporation and Capto Core 700 column purification on luciferase expression (all positive controls are treatment groups of 0.375 µg/ml liraglutide without electroporation).
FIG. 15 shows the conditions for exploring the use of incubation to load PKH67 in exosomes; wherein, FIG. 15A shows the FITC positive rate and the particle concentration of the samples after incubation of the PKH67-exosome mixture at different temperatures (0.5 h); and FIG. 15B shows the FITC positive rate and the particle concentration of the samples after incubation at 50°C for different periods of time.
FIG. 16 shows the result obtained from Capto Core 700 column purification of electroporated liraglutide.
FIG. 17 shows the effects of the liraglutide solution and the exosome-liraglutide mixture after co-incubation and Capto Core 700 column purification on luciferase expression (all positive controls are treatment groups of 0.375 µg/ml liraglutide without co-incubation).
FIG. 18 shows the cellular efficacy of exosomes loaded with different drugs.
FIG. 19 shows the particle number of positive exosomes in serum of mice after administration by gavage at different time points as detected by nanoflow cytometry.
FIG. 20 shows the particle number of positive exosomes in serum of mice after sublingual administration at different time points as detected by nanoflow cytometry.
FIG. 21 shows changes over time in blood glucose of mice sublingually administered with exosomes loaded with different drugs and given glucose solution by gavage after 1 h.
FIG. 22 shows the changes over time in random blood glucose of mice sublingually administered with different concentrations of exosome-liraglutide.
FIG. 23 shows the changes over time in fasting blood glucose of mice sublingually administered with different concentrations of exosome-liraglutide after 6 h of fasting.

### DETAILED DESCRIPTION

The present disclosure discloses use of a milk exosome in the preparation of a drug carrier. Those skilled in the art can learn from the contents of the disclosure and appropriately adapt the process parameters to achieve the present disclosure. Particularly, it should be noted that all such alternatives and modifications are obvious to those skilled in the art and are considered to be included within the present disclosure. The method and the application of the present disclosure have been described through the preferred embodiments, and it is obvious that the method and application described herein may be changed or appropriately modified and combined to realize and apply the technology of the present disclosure by those skilled in the art without departing from the content, spirit and scope of the present disclosure.

All materials and reagents used in the use of a milk exosome in the preparation of a drug carrier provided by the present disclosure are commercially available.

The present disclosure is further illustrated below in combination with the embodiments.

### Example 1 Industrially scalable purification process for milk exosomes

### 1) Pre-treatment of milk samples by different protein removal methods

a. EDTA precipitation method: Skimmed milk was mixed with EDTA of the same volume, different concentrations and different pHs, and incubated at room temperature for 15 min to precipitate casein. The EDTA used had a concentration of 0.15-0.35 mol·L⁻¹ and a pH of 6.0-8.0. Centrifugation was carried out at 12,000 r·min⁻¹ for 40 min, and the supernatant was filtered through 0.80, 0.45 and 0.20 µm membrane filters sequentially.
b. Sodium phosphate precipitation method: An equal amount of skimmed milk was added to a sodium phosphate solution. Several pre-tests were carried out on the concentration and pH range of sodium phosphate, such as using 0.5 mol·L⁻¹ (pH 5.3, pH 6.5 and pH 7.6), 1.0 mol·L⁻¹ (pH 5.2, pH 6.3 and pH 7.6) and 2 mol·L⁻¹ (pH 6.0, pH 7.0 and pH 8.0) sodium phosphate. This mixture was placed at 4°C overnight to precipitate protein, and the supernatant after precipitation was sequentially filtered through 0.80, 0.45, and 0.20 µm membrane filters.
c. Sodium citrate dissolution method: skimmed milk was mixed with equal amounts of 2%, 4%, 6%, 8% and 16% sodium citrate, incubated on ice for 2 h to dissolve casein, and filtered through a 0.20 µm membrane filter.

### 2) Preliminary purification and concentration of milk exosomes by TFF method

The milk supernatant after pre-treatment was processed using a hollow fiber column with a relative molecular mass of 750,000. The system parameters were set as follows: TMP maximum pressure at 2 atmospheres, alarm pressure at 2 atmospheres; 100 mL minimum volume, and 100 mL alarm volume. The sample was poured into a reusable beaker, and exchanged with 1×PBS until the concentration of permeate proteins was 0.00 g·L⁻¹. Then the liquid in the beaker was further concentrated to 1/20 of the original solution, and the sample was collected.

### 3) Downstream purification of milk exosomes by BE-SEC and UC

The ÄKTA Pure 25 purification system was rinsed with ultrapure water, connected to a HiScreen Capto Core 700 column, and then rinsed with 3 column volumes of ultrapure water. After rinsing, the equilibration was carried out with 3 column volumes of 1×PBS, and the preliminarily purified sample was purified using a HiScreen Capto Core 700 column at a flow rate of 2 mL·min⁻¹. The sample was collected at the position of the flow-through peak, i.e. milk exosomes.

### Experimental results

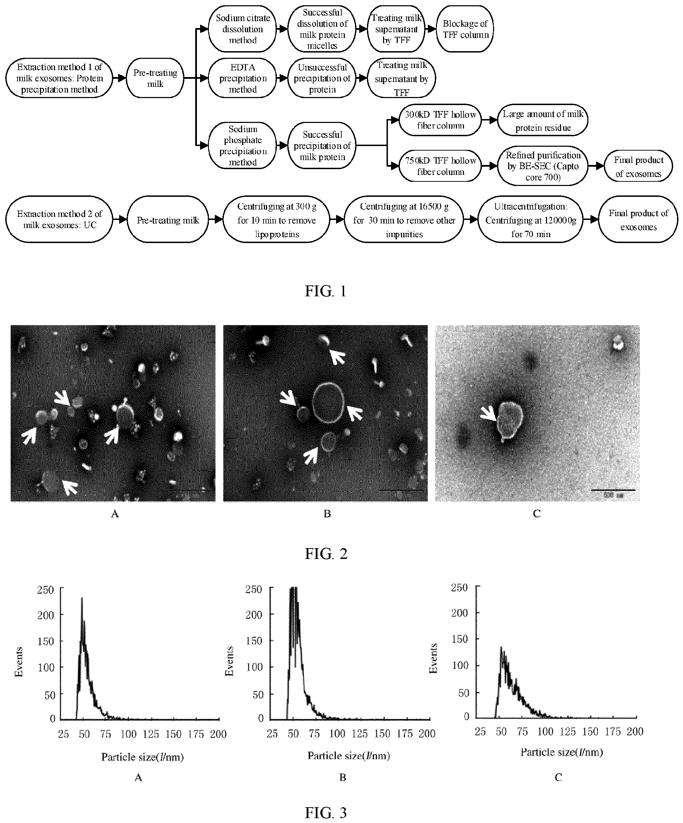

As shown in FIG. 1, the protein precipitation method was used to obtain a pretreated sample, and the purification method TFF in combination with BE-SEC was used to obtain milk exosomes. It was demonstrated that the sample obtained by this process has a higher yield and purity compared with UC, a traditional extraction method of exosomes.

As shown in FIG. 2, the membrane structures of exosomes extracted by TFF, BE-SEC and UC can all be observed under TEM, showing a saucer-like or disc-like shape. However, the exosomes extracted by UC under TEM had a cluttered background and more protein aggregates. In comparison, exosomes purified by TFF combined with BE-SEC had a cleaner background.

The particle size, concentration and purity of exosomes detected by NanoFCM are shown in FIG. 3. The exosomes obtained by TFF had the average particle size of (66.92±16. 39) nm, a yield of 3.6×10¹³ particles·L⁻¹ and a purity of 58%. The milk exosomes prepared by TFF+BE-SEC had the diameter concentrated in a range of 30-150 nm, the average particle of (66.41±15.81) nm, a yield of 2.5×10¹³ particles·L⁻¹ and a purity of 90%. The exosomes obtained by UC had the average particle size of (68.87±18.04) nm, a yield of 2.4×10¹¹ particles·L⁻¹ and a purity of 25%. It was demonstrated that the exosomes prepared by TFF+BE-SEC had a higher yield and purity.

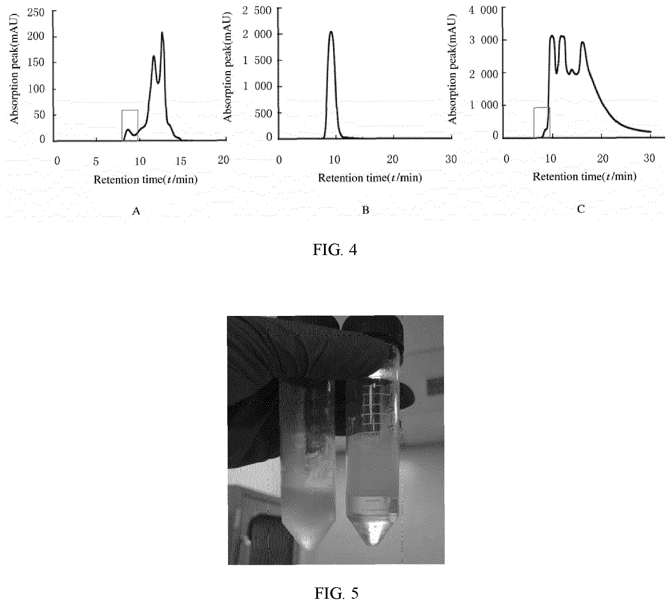

As shown in FIG. 4, the amount of free proteins (particle size smaller than that of exosomes) in the exosome sample was detected by HPLC. The retention time of milk exosomes extracted by the three methods on a TSKgel G6000 PWXL column was all 8 min. However, the proportion of impurity peaks (RT>8 min) of the exosome samples extracted by TFF and BE-SEC was smaller than that of the samples obtained by UC, and the final product obtained by TFF+BE-SEC showed more of a single peak.

The use of protein precipitation pre-treatment in combination with purification by TFF and BE-SEC of milk can obtain exosome samples with a higher yield and purity. More importantly, TFF could achieve the preliminary purification and concentration of samples from a few liters to hundreds of liters, and the large-scale preparation of milk exosomes could finally be achieved by combining TFF with BE-SEC.

### Example 2 Drug loading process for milk exosomes - ultrasonication method

Principle: Ultrasonication can disrupt the arrangement of phospholipid bilayer in the membrane and create temporary pores. After exosomes are subjected to low-frequency continuous ultrasonication, the sound wave causes the encapsulated air to expand and the pressure inside the vesicle to rise to the elastic limit of the bilayer, resulting in pores in the phospholipid layer. Due to the high drug concentration outside the membrane, the difference in osmotic pressure causes the drug to diffuse into the liposome through the pores.

### Operation steps

The optimal conditions for loading by ultrasonication were explored using PKH67. Specifically, after co-incubation of PKH67 with milk exosomes at room temperature for 30 min, ultrasonication was carried out using a cell crusher at the power of 150w, 300w, 450w, 600w, 800w, 1000w and 1200w for 0 s, 50 s, 250 s, 500 s, 750 s, 20 min, 30 min, 1 h, 2 h, 4 h and 6 h. The particle number and PKH67 positive rate of the ultrasonicated samples were determined by using NanoFCM. Based on the optimal conditions for loading by ultrasonication, the volume for loading by ultrasonication was explored. The particle number, particle size and PKH67 positive rate of the ultrasonicated samples were determined by using NanoFCM.

### Experimental results

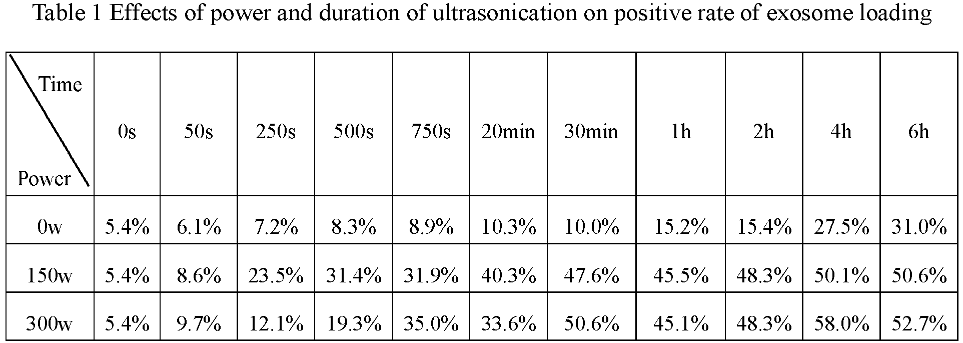

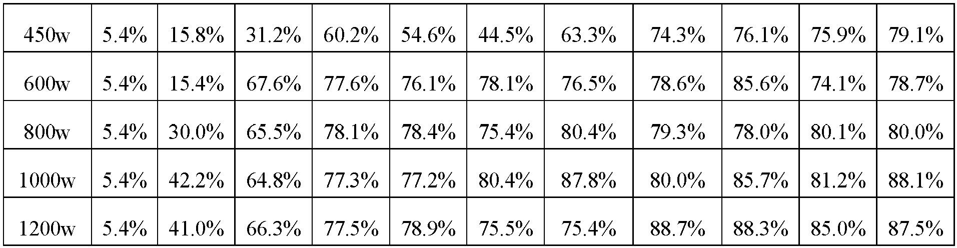

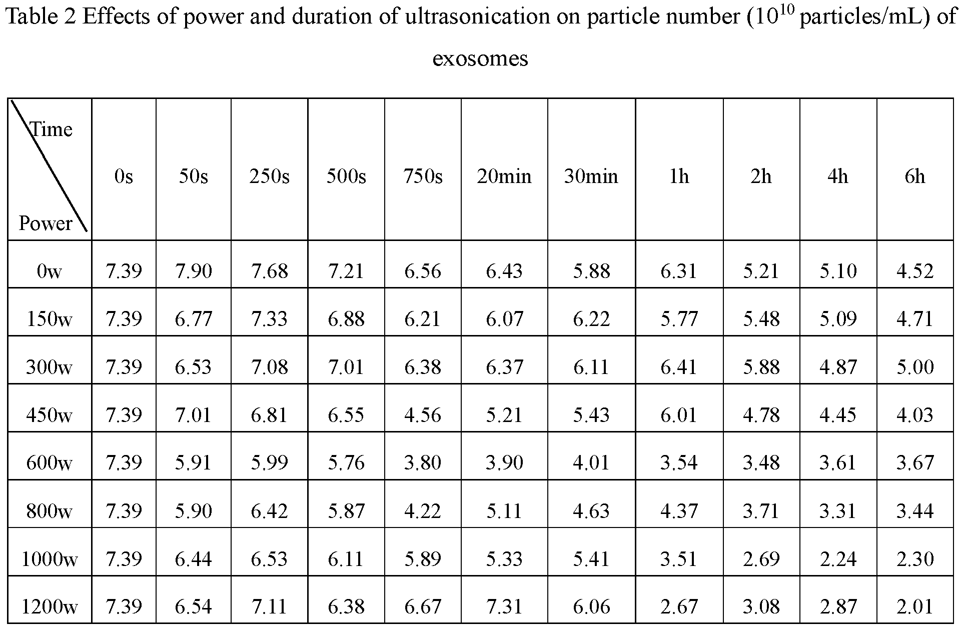

**Table 3 Effects of different volumes for ultrasonication (600w, 500s) on exosome loading**

| Volume (ml) | Positive rate (%) | Particle concentration (10¹⁰ particles/mL) |
|---|---|---|
| 3 | 77.8 | 5.23 |
| 10 | 75.3 | 5.60 |
| 50 | 80.1 | 4.85 |
| 100 | 78.1 | 5.08 |
| 200 | 75.6 | 4.66 |
| 500 | 74.7 | 4.97 |

There was a large difference in exosome loading efficiency after ultrasonication at different power, and the positive rate after ultrasound was between 5% and 90%. For ultrasonication at 450W-1200W power for more than 20 min, the difference in loading efficiency (as gauged by the positive rates, see Table 1) was not obvious and was mostly around 80%. With longer ultrasonication time, the particle number decreased. The loading scale of 3 ml-500 ml system could be achieved, and the particle number and loading positive rate after loading were relatively stable.

### Loading liraglutide by ultrasonication:

After mixing according to the ratio of 1×10¹¹ exosome particles to 5 mg liraglutide, ultrasonication was performed followed by purification using Capto Core 700 or QA columns. There was an obvious change in the state of the solution before and after ultrasonication. FIG. 5 shows a liraglutide solution after ultrasonication (left: a colloidal solution with more precipitates after ultrasonication) and a mixture of milk exosomes and liraglutide (right).

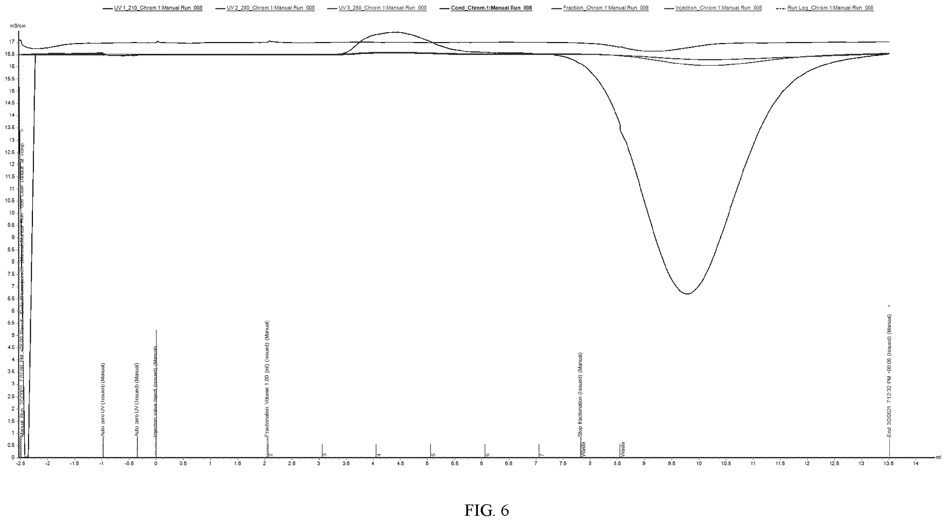

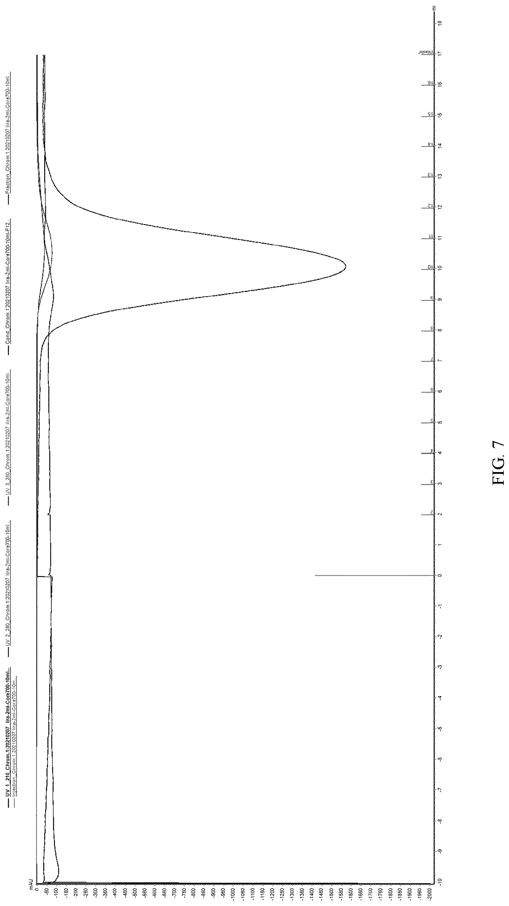

25 mg of liraglutide, and a mixture of 25 mg liraglutide and 5×10¹¹ exosome particles were ultrasonicated and then passed through a Capto Core 700 column to collect flow-through samples. The results are shown in FIGs. 6-7. On a 5 mL Capto Core 700 chromatography column, 2-4 mL of flow-through sample was collected after the injection started. This fraction was the exosome particles loaded with liraglutide, and the free liraglutide was adsorbed on the column and then removed by elution.

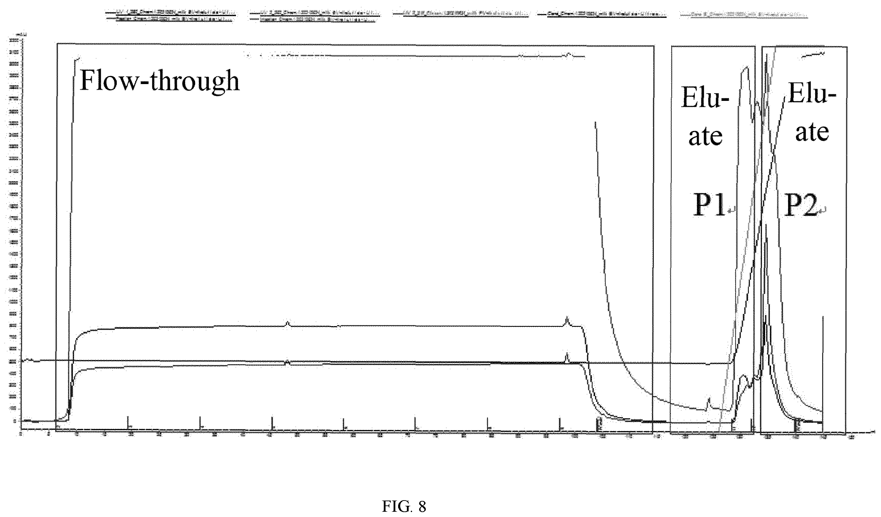

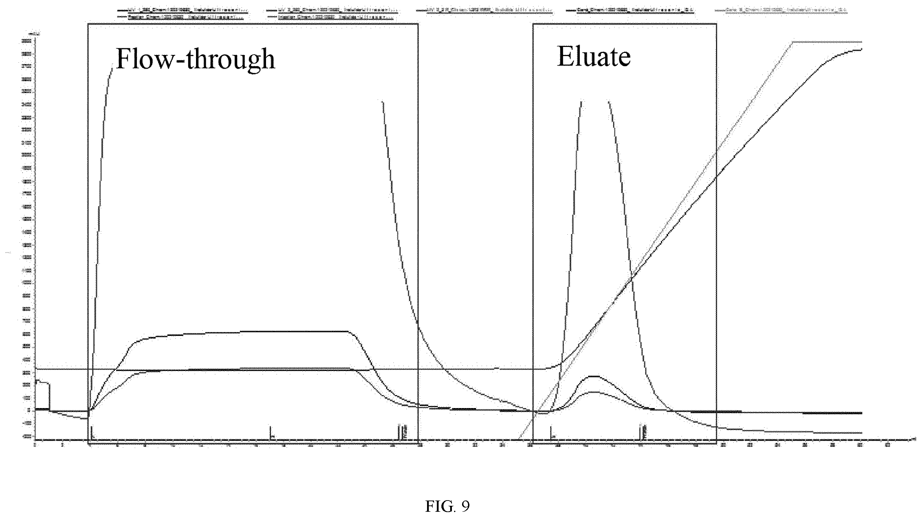

250 mg liraglutide, and a mixture of 250 mg liraglutide and 5×10¹² exosome particles were ultrasonicated and then passed through a QA column for purification, and flow-through and eluted samples were collected. The results are shown in FIGs 8-9. The liraglutide-loaded exosomes and free liraglutide were eluted separately under different salt concentrations based on the difference in electrostatic charges.

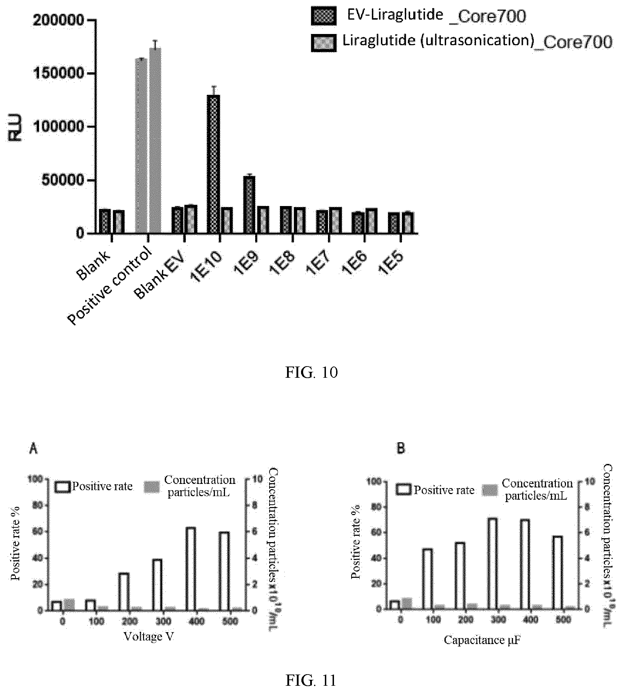

GLP-1R-CHO cells were seeded into a 96-well plate and cultured for 24 h, and then the medium was replaced with blank milk exosomes, milk exosome-liraglutide (10⁵/mL, 10⁶/mL, 10⁷/mL, 10⁸/mL, 10⁹/mL and 10¹⁰/mL) or liraglutide solutions with the corresponding particle concentration for 5 h of culture. The samples were collected according to the instructions of luciferase kit, and 100 µl of cell lysate was added to each well to lyse cells for 5 min. The cell lysate was collected and centrifuged at 1200 rpm for 5 min. Then the substrate was added and incubated for 5 min, and the fluorescence was detected using an enzyme reader. The results are shown in FIG. 10 and Table 4.

**Table 4**

| | Exosome-liraglutide_Capto Core 700 | | | Liraglutide (ultrasonication)_Capto Core 700 | | |
|---|---|---|---|---|---|---|
| Blank | 20617 | 19685 | 22845 | 19513 | 20557 | 21774 |
| Positive control | 160126 | 161819 | 164485 | 166442 | 168105 | 182139 |
| Blank exosomes | 21244 | 24053 | 24830 | 22927 | 25572 | 26806 |
| 1×10¹⁰ | 117775 | 129093 | 137324 | 23620 | 23463 | 23987 |
| 1×10⁹ | 49581 | 52138 | 56060 | 22659 | 23782 | 25679 |
| 1×10⁸ | 24063 | 25156 | 24889 | 24413 | 23676 | 23367 |
| 1×10⁷ | 20066 | 20339 | 22143 | 23953 | 23706 | 24012 |
| 1×10⁶ | 17023 | 18083 | 20867 | 22732 | 22735 | 22400 |
| 1×10⁵ | 18999 | 17390 | 18551 | 17634 | 17430 | 21039 |

Experimental conclusion: From the comparison of the liquid appearance of liraglutide with that of the milk exosome-liraglutide mixture after ultrasonication, it can be seen that liraglutide did not precipitate when containing exosomes, but was loaded on the exosomes. After ultrasonication and QA purification of the milk exosome-liraglutide mixture, the chromatogram showed that the A260/A280 absorption ratio of the samples after loading by ultrasonication in high salt elution (conductivity of 70.44 mS/cm) was increased to 1.88, which represented the absorption peak of nucleic acid contents released from exosomes of which the bilayer membrane was damaged by ultrasonication. The particle number of exosomes decreased after prolonged ultrasound time. After purification through Capto Core 700 or QA columns, the drug-treated cells were proportionally diluted, and it was shown that the expression of luciferase was dose-dependent. The treatment of 1×10¹⁰ particles of milk exosome-exosome-liraglutide on cells significant increased luciferase expression, indicating that the loading of liraglutide in milk exosomes was achieved with biological activity.

### Example 3 Drug loading process for milk exosomes - electroporation method

Principle: Electroporation is a microbiological technique, where an electric field is applied to a cell to increase the permeability of the cell membrane, thereby allowing chemicals, drugs or DNA to be introduced into the cell.

### Operation steps:

The optimal conditions for drug loading by electroporation were explored using PKH67. Specifically, after co-incubation of PKH67 with milk exosomes at room temperature for 30 min, electroporation was carried out using an electroporator at a voltage of 100-500 V and a capacitance of 100-500 µF. The particle number, particle size and PKH67 positive rate of the electroporated samples were determined by using NanoFCM.

25 mg of liraglutide, and a mixture of 25 mg liraglutide and 5×10¹¹ exosome particles were electroporated and then passed through a Capto Core 700 column to remove free liraglutide. The flow-through samples were collected.

GLP-1R-CHO cells were seeded into a 96-well plate and cultured for 24 h, and then the medium was replaced with blank milk exosomes, milk exosome-liraglutide (10⁵/mL, 10⁶/mL, 10⁷/mL, 10⁸/mL, 10⁹/mL and 10¹⁰/mL) or liraglutide solutions with the corresponding particle concentration for 5 h of culture. The samples were collected according to the instructions of luciferase kit, and 100 µl of cell lysate was added to each well to lyse cells for 5 min. The cell lysate was collected and centrifuged at 1200 rpm for 5 min. Then the substrate was added and incubated for 5 min, and the fluorescence was detected using an enzyme reader.

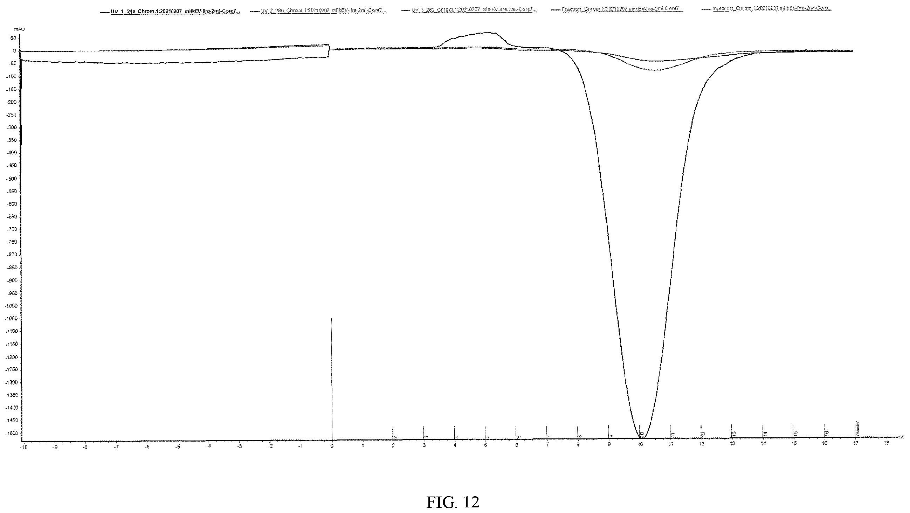

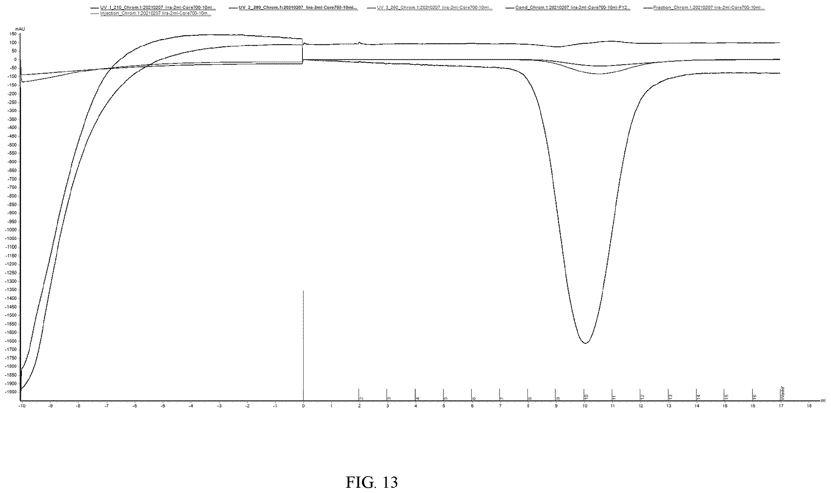

The results are shown in FIGs. 11-14 and Tables 5-7.

**Table 5 Effect of voltage on exosomes loading**

| Voltage V | Positive rate % | Concentration particles×10¹⁰/mL |
|---|---|---|
| 0 | 7.12 | 8.55 |
| 100 | 8.2 | 2.8 |
| 200 | 28.5 | 2.52 |
| 300 | 39 | 2.26 |
| 400 | 62.9 | 1.56 |
| 500 | 59.5 | 2.14 |

**Table 6 Effect of capacitance on exosomes loading**

| Capacitance µF | Positive rate % | Concentration particles×10¹⁰/mL |
|---|---|---|
| 0 | 6.35 | 8.55 |
| 100 | 46.8 | 3.2 |
| 200 | 51.8 | 3.78 |
| 300 | 71.2 | 2.78 |
| 400 | 69.8 | 2.96 |
| 500 | 57.1 | 2.11 |

**Table 7**

| | Exosome-liraglutide_Capto Core 700 | | | Liraglutide (electroporation)_Capto Core 700 | | |
|---|---|---|---|---|---|---|
| Blank | 16617 | 15685 | 21845 | 17513 | 18557 | 19774 |
| Positive control | 170126 | 131819 | 144485 | 196442 | 128105 | 122139 |
| Blank exosomes | 18244 | 20053 | 24830 | 22927 | 18572 | 20806 |
| 1×10¹⁰ | 97775 | 92093 | 87324 | 20620 | 20463 | 20987 |
| 1×10⁹ | 50581 | 42138 | 46060 | 21659 | 22782 | 18679 |
| 1×10⁸ | 22063 | 22156 | 22889 | 19413 | 18676 | 20367 |
| 1×10⁷ | 20066 | 20339 | 22143 | 23953 | 23706 | 24012 |
| 1×10⁶ | 17023 | 18083 | 20867 | 22732 | 22735 | 22400 |
| 1×10⁵ | 18999 | 17390 | 18551 | 17634 | 17430 | 21039 |

### Example 4 Drug loading process for milk exosomes - co-incubation method

Principle: The drug will be adsorbed on the drug carrier by the interaction between substances.

### Operation steps

The optimal conditions for loading by co-incubation were explored using PKH67. Specifically, after co-incubation of PKH67 with milk exosomes at room temperature for 30 min, the samples were incubated at 4°C, 37°C and 50°C with shaking at 500 rpm for 30 min, 1 h, and 2 h. The particle number, particle size and PKH67 positive rate of the co-incubated samples were determined by using NanoFCM.

25 mg of liraglutide, and a mixture of 25 mg liraglutide and 5×10¹¹ exosome particles were co-incubated and then passed through a Capto Core 700 column to remove free liraglutide. The flow-through samples were collected.

GLP-1R-CHO cells were seeded into a 96-well plate and cultured for 24 h, and then the medium was replaced with blank milk exosomes, milk exosome-liraglutide (10⁵/mL, 10⁶/mL, 10⁷/mL, 10⁸/mL, 10⁹/mL and 10¹⁰/mL) or liraglutide solutions with the corresponding particle concentration for 5 h of culture. The samples were collected according to the instructions of luciferase kit, and 100 µl of cell lysate was added to each well to lyse cells for 5 min. The cell lysate was collected and centrifuged at 1200 rpm for 5 min. Then the substrate was added and incubated for 5 min, and the fluorescence was detected using an enzyme reader.

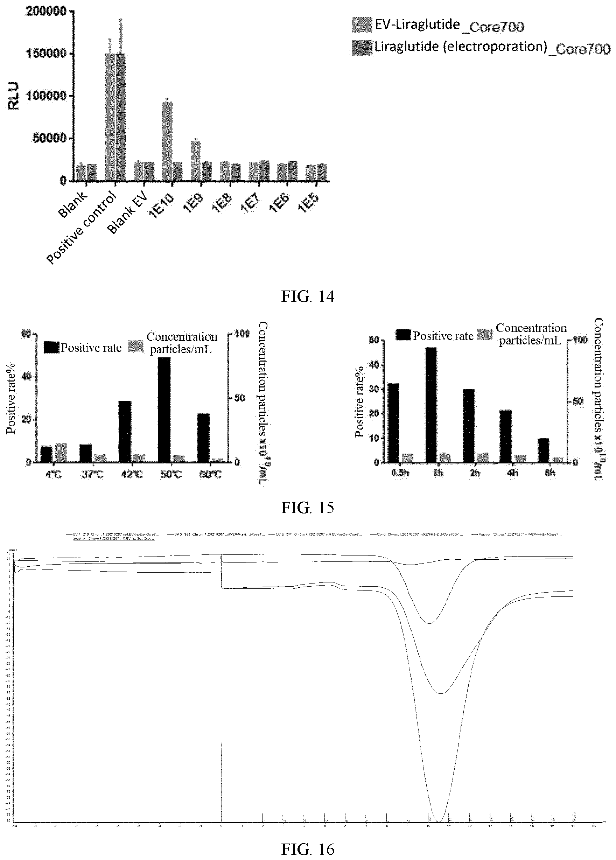

The results are shown in FIGs. 14-17 and Tables 8-10.

**Table 8 Effect of incubation temperature on exosome loading**

| Temperature | Positive rate % | Concentration particles×10¹⁰/mL |
|---|---|---|
| 4°C | 7.12 | 8.55 |
| 37°C | 8.2 | 3.18 |
| 42°C | 28.5 | 3.32 |
| 50°C | 49 | 3.26 |
| 60°C | 22.9 | 1.56 |

**Table 9 Effect of incubation time on exosome loading**

| Time | Positive rate % | Concentration particles×10¹⁰/mL |
|---|---|---|
| 0.5h | 32.1 | 3.6 |
| 1h | 46.8 | 3.78 |
| 2h | 29.8 | 3.78 |
| 4h | 21.2 | 2.78 |
| 8h | 9.8 | 1.96 |

**Table 10 Cellular efficacy by co-incubation method**

| | Exosome-liraglutide_Capto Core 700 | | | Liraglutide (incubation)_Capto Core 700 | | |
|---|---|---|---|---|---|---|
| Blank | 16617 | 15685 | 21845 | 17513 | 18557 | 19774 |
| Positive control | 190126 | 131819 | 144485 | 156442 | 128105 | 122139 |
| Blank exosomes | 18244 | 20053 | 24830 | 22927 | 18572 | 20806 |
| 1×10¹⁰ | 77775 | 72093 | 87324 | 14620 | 15463 | 13987 |
| 1×10⁹ | 40581 | 32138 | 46060 | 11659 | 12782 | 11679 |
| 1×10⁸ | 12063 | 12156 | 12889 | 9413 | 8676 | 80367 |
| 1×10⁷ | 10066 | 10339 | 12143 | 7953 | 7306 | 74012 |
| 1×10⁶ | 7023 | 8083 | 7867 | 5732 | 6735 | 6400 |
| 1×10⁵ | 6999 | 7390 | 6551 | 4634 | 5430 | 4039 |

Experimental conclusion: After purification through Capto Core 700, the drug-treated cells were proportionally diluted, and it was shown that the expression of luciferase was dose-dependent. The treatment of 1×10¹⁰ particles of milk exosome-liraglutide on cells significant increased luciferase expression, indicating that the loading of liraglutide in milk exosomes was achieved with biological activity.

### Example 5 Loading different T2DM drugs in milk exosomes

At present, there are many drugs for treating type 2 diabetes mellitus (T2DM) on the market, such as insulin degludec, insulin detemir, liraglutide, semaglutide, and tirzepatide which has been listed abroad. The present disclosure realized loading different T2DM drugs in milk exosomes, so that these drugs can be administered orally instead of by injection, solving the dilemma of no oral diabetes drugs on the market.

### Operation

1) According to the drug loading method of milk exosomes introduced in 4.2, the electroporation method was selected. The milk exosomes were loaded with insulin degludec, insulin detemir, liraglutide, semaglutide and tirzepatide separately, and purified through a Capto Core 700 column.

2) GLP-1R-CHO cells were seeded into a 96-well plate and cultured for 24 h, and then the medium was replaced with blank milk exosomes, or milk exosome loaded with different drugs (10⁵/mL, 10⁶/mL, 10⁷/mL, 10⁸/mL, 10⁹/mL and 10¹⁰/mL) for 5 h of culture. The samples were collected according to the instructions of luciferase kit, and 100 µl of cell lysate was added to each well to lyse cells for 5 min. The cell lysate was collected and centrifuged at 1200 rpm for 5 min. Then the substrate was added and incubated for 5 min, and the fluorescence was detected using an enzyme reader.

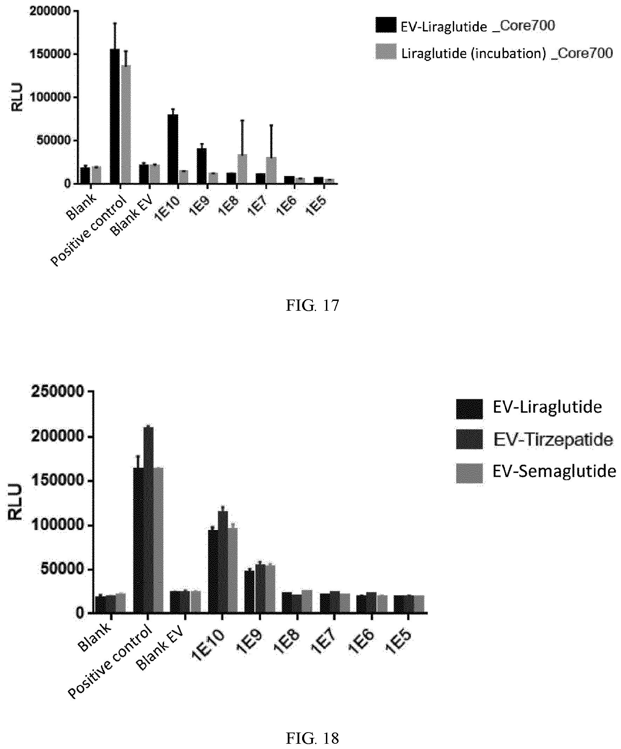

The results are shown in FIG. 18 and Table 11.

**Table 11 Cellular efficacy of exosomes loaded with different drugs**

| | Exosmome-liraglutide | | | Exosome-tirzepatide | | | Exosome-semaglutide | | |
|---|---|---|---|---|---|---|---|---|---|
| Blank | 16617 | 15685 | 21845 | 17513 | 18557 | 19774 | 20617 | 19685 | 22845 |
| Positive control | 170126 | 171819 | 144485 | 206442 | 208105 | 212139 | 160126 | 161819 | 164485 |
| Blank exosomes | 23244 | 24053 | 24830 | 22927 | 26572 | 20806 | 21244 | 24053 | 24830 |
| 1×10¹⁰ | 97775 | 92093 | 87324 | 110620 | 110463 | 120987 | 97775 | 99093 | 87324 |
| 1×10⁹ | 50581 | 42138 | 46060 | 51659 | 52782 | 58679 | 49581 | 52138 | 56060 |
| 1×10⁸ | 22063 | 22156 | 22889 | 19413 | 18676 | 20367 | 24063 | 25156 | 24889 |
| 1×10⁷ | 20066 | 20339 | 22143 | 23953 | 23706 | 24012 | 20066 | 20339 | 22143 |
| 1×10⁶ | 17023 | 18083 | 20867 | 22732 | 22735 | 22400 | 17023 | 18083 | 20867 |
| 1×10⁵ | 18999 | 17390 | 18551 | 17634 | 17430 | 21039 | 18999 | 17390 | 18551 |

### Experimental conclusion:

From the results, milk exosomes loaded with different drugs enable cells to release GLP in a dose-dependent manner, indicating that the present disclosure can realize the loading of different types of protein and polypeptide drugs.

### Example 6 Sublingual administration of fluorescently labeled milk exosomes

Principle: Utilizing the characteristics of the sublingual mucosa that is not keratinized, has abundant capillaries, and has fast blood flow speed, and aiming to solve problems that milk exosomes are easily destroyed by gastric acid after passing through the gastrointestinal tract, resulting in low bioavailability of the loaded drug, the present disclosure prepares liraglutide-loaded milk exosomes into sublingual tablets to achieve oral administration of liraglutide, which can be absorbed through the sublingual mucosa and directly enter the blood circulation through the jugular vein and superior vena cava, with a rapid onset of action. In addition, the drug can be taken without water by placing it under the tongue to dissolve, which is convenient to take and has a good taste.

### Experimental operation:

A. PKH67-labelled milk exosomes were administered via 1) gavage, 2) sublingually, and 3) as enteric capsules.
B. After administration, blood was collected from mice at different time points, and the number of positive particles in the blood was detected by NanoFCM to determine the absorption efficiency of exosomes into the blood.

For PKH67-labelled milk exosomes administered to mice by gavage, the blood was collected at different time points to detect PKH67-positive particles and 4×10¹¹ particles were administered per mouse. After 5 min, the maximum number of PKH67-posivie particles were detected in the blood of the mice (2×10⁹), corresponding to 0.5% exosomes entering the blood. For PKH67-labelled milk exosomes administered sublingually, the maximum number of PHK67-positive particles detected in the blood (also at 5min) was 2×10¹⁰, corresponding to 5% exosomes entering the blood. For administration by enteric capsules, the number of PKH67-positive particles in the blood of mice was not detected. Therefore, the bioavailability by sublingual administration is significantly higher compared to administration by gavage.

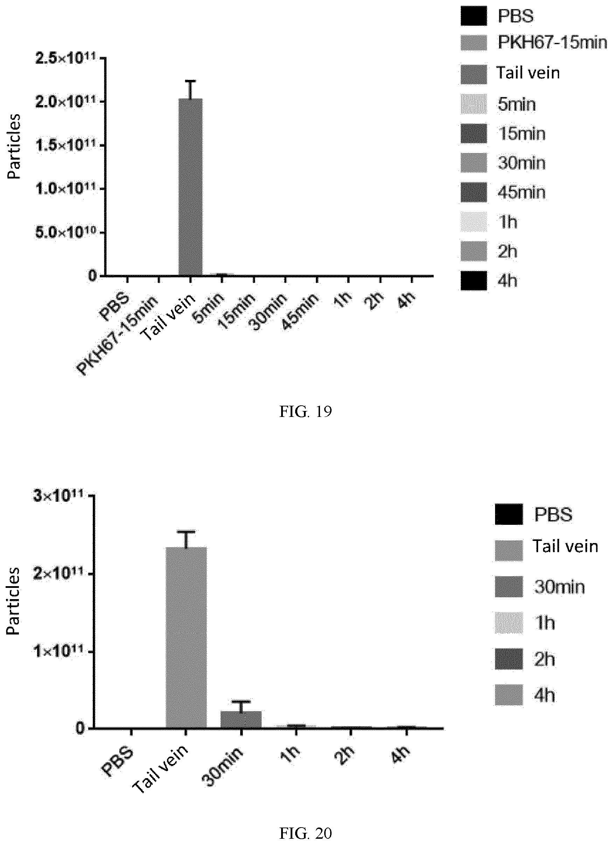

The experimental results are shown in FIGs. 19-20 and Tables 12-14.

**Table 12 Data from administration by gavage**

| Mode of administration | PKH67 Positive particles | | | | | |
|---|---|---|---|---|---|---|
| PBS | 8.70×10⁷ | 1.74×10⁷ | 6.46×10⁷ | 3.57×10⁷ | 4.29×10⁷ | 6.83×10⁷ |
| PKH67-15min | 3.30×10⁸ | 2.44×10⁸ | 2.01×10⁸ | 2.09×10⁸ | 3.04×10⁸ | 9.87×10⁷ |
| Tail vein | 1.68×10¹¹ | 2.34×10¹¹ | 2.12×10¹¹ | 1.98×10¹¹ | 2.01×10¹¹ | 2.05×10¹¹ |
| 5min | 1.85×10⁹ | 2.32×10⁹ | 1.94×10⁹ | 2.05×10⁹ | 1.77×10⁹ | 2.11×10⁹ |
| 15min | 7.48×10⁸ | 3.66×10⁸ | 6.96×10⁸ | 4.87×10⁸ | 6.12×10⁸ | 5.34×10⁸ |
| 30min | 4.00×10⁸ | 2.62×10⁸ | 1.57×10⁸ | 3.56×10⁸ | 1.22×10⁸ | 3.19×10⁸ |
| 45min | 3.30×10⁸ | 2.26×10⁸ | 1.22×10⁸ | 2.98×10⁸ | 3.09×10⁸ | 1.78×10⁸ |
| 1h | 2.44×10⁸ | 2.08×10⁸ | 1.74×10⁸ | 1.35×10⁸ | 9.98×10⁷ | 2.04×10⁸ |
| 2h | 1.57×10⁸ | 2.78×10⁸ | 1.39×10⁸ | 1.34×10⁸ | 1.24×10⁸ | 8.99×10⁷ |
| 4h | 1.39×10⁸ | 1.04×10⁸ | 1.57×10⁸ | 1.02×10⁸ | 1.11×10⁸ | 8.34×10⁷ |

**Table 13 Data from sublingual administration**

| Mode of administration | PKH67 Positive particles | | | | | |
|---|---|---|---|---|---|---|
| PBS | 6.60×10⁷ | 6.60×10⁷ | 5.89×10⁷ | 6.01×10⁷ | 5.46×10⁷ | 5.28×10⁷ |
| Tail vein | 2.60×10¹¹ | 2.30×10¹¹ | 2.45×10¹¹ | 1.98×10¹¹ | 2.45×10¹¹ | 2.19×10¹¹ |
| 30min | 7.88×10⁹ | 5.78×10⁹ | 6.56×10⁹ | 2.89×10¹⁰ | 4.02×10¹⁰ | 3.27×10¹⁰ |
| 1h | 3.50×10⁹ | 5.08×10⁹ | 1.04×10⁹ | 7.54×10⁸ | 4.78×10⁹ | 1.67×10⁹ |
| 2h | 1.11×10⁹ | 5.36×10⁸ | 8.97×10⁸ | 7.29×10⁸ | 1.02×10⁹ | 6.92×10⁸ |
| 4h | 4.26×10⁸ | 4.72×10⁸ | 4.72×10⁸ | 6.30×10⁸ | 2.78×10⁹ | 3.10×10⁹ |

**Table 14 Data of enteric capsules**

| | mean | SD |
|---|---|---|
| 0min | 4.58×10⁷ | 2.67×10⁷ |
| 30min | 6.10×10⁷ | 1.89×10⁷ |
| 1h | 1.53×10⁷ | 9.87×10⁶ |
| 2h | 1.53×10⁷ | 8.22×10⁶ |
| 3h | 3.05×10⁷ | 1.24×10⁷ |
| 4h | 6.86×10⁷ | 2.78×10⁷ |
| 5h | 1.30×10⁸ | 9.18×10⁶ |
| 8h | 1.53×10⁷ | 7.35×10⁶ |
| 22h | 9.92×10⁷ | 3.22×10⁷ |

### Example 7 Sublingual tablets of milk exosomes loaded with Liraglutide

Experimental operation: Lyophilized powder of milk exosomes loaded with liraglutide was mixed with a disintegrant, filler, binder, lubricant and flavoring agent to prepare into sublingual tablets. In each 1,000 tablets of sublingual tablets of liraglutide milk exosomes, the drug-loaded exosome has a content of 10⁵-10¹⁷ particles, the disintegrating agent 1-50 g, the filler 20-400g, the flavoring agent 0-50 g, the binder 5-100 g, and the lubricant 1 g-10 g;

The disintegrating agent used was selected from the group consisting of microcrystalline cellulose, cross-linked sodium carboxymethyl cellulose, cross-linked polyvinylpyrrolidone, sodium carboxymethyl starch, low-substituted hydroxypropyl cellulose, starch, Tween, sodium dodecyl sulfate, and a mixture thereof.

The filler used was selected from the group consisting of microcrystalline cellulose, microcrystalline cellulose-mannitol, microcrystalline cellulose-micronized silica gel, lactose, starch, modified starch, mannitol, sorbitol, xylitol, erythrose, trehalose, pregelatinized starch, icing sugar, glucose, dextrin, calcium sulfate, and a mixture thereof.

The flavoring agent used was selected from the group consisting of stevioside, icing sugar, glycyrrhizin, aspartame, sucralose, cyclamate, thaumatin, saccharin, and a mixture thereof.

The binder used was selected from the group consisting of purified water, starch slurry, hydroxypropyl methylcellulose, polyvinylpyrrolidone, carbomer, dextrin, gelatin slurry, gum arabic slurry, sodium alginate, syrup, and a mixture thereof.

The lubricant used was selected from the group consisting of stearic acid, magnesium stearate, calcium stearate, micronized silica gel, talc, hydrogenated vegetable oil, polyethylene glycol 4000, polyethylene glycol 6000, sodium dodecyl sulfate, magnesium dodecyl sulfate, sodium stearyl fumarate, and a mixture thereof.

The optimal formula is as follows: 1×10¹⁵ particles of exosome-liraglutide, 4 g cross-linked sodium carboxymethyl cellulose, 80 g mannitol, 25 g purified water and 3.5 g magnesium stearate. The preparation was performed according to the following preparation steps.

### Preparation steps:

Step 1: The drug-loaded exosome was lyophilized and passed through 80-120 mesh sieves.
Step 2: The filler, disintegrating agent, flavoring agent and lubricant were dried, crushed and passed through 80-120 mesh sieves for pre-treatment.
Step 3: The pre-treated drug-loaded exosome, filler, flavoring agent and disintegrating agent were mixed well.
Step 4: The binder was added to the above well-mixed materials to prepare soft materials, which were then granulated and dried.
Step 5: The lubricant was added to the dry granules, mixed well and then pressed to obtain the final product.

### Example 8 An improved formulation for the sublingual tablets of milk exosomes loaded with Liraglutide

1×10¹³ particles of the drug-loaded exosome, was added to 0.6 g mannitol, lyophilized, dried with 55 g microcrystalline cellulose the filler, 1 g sodium carboxymethyl cellulose the disintegrant, and 1 g magnesium stearate the lubricant, crushed, sieved, mixed, prepared into a soft material, granulated with a 20 mesh sieve, air dried to moisture≤3%, then added with lubricant for final blending, and finally pressed to obtain sublingual tablets of liraglutide-milk exosomes.

### Example 9 Sublingual tablets of milk exosomes, loaded with T2DM treatment drugs

### Experimental operation

By electroporation method, insulin degludec, insulin detemir, liraglutide, semaglutide, and tirzepatide were loaded into milk exosomes and purified to obtain drug-loaded exosomes, which were prepared into sublingual tablets according to the formula in Example 8.

Each mouse was sublingually administered with sublingual tablets containing 1×10¹¹ particles of drug-loaded milk exosomes. The animal were evaluated for their tolerance of elevated blood glucose level (induced via injection of glucose).

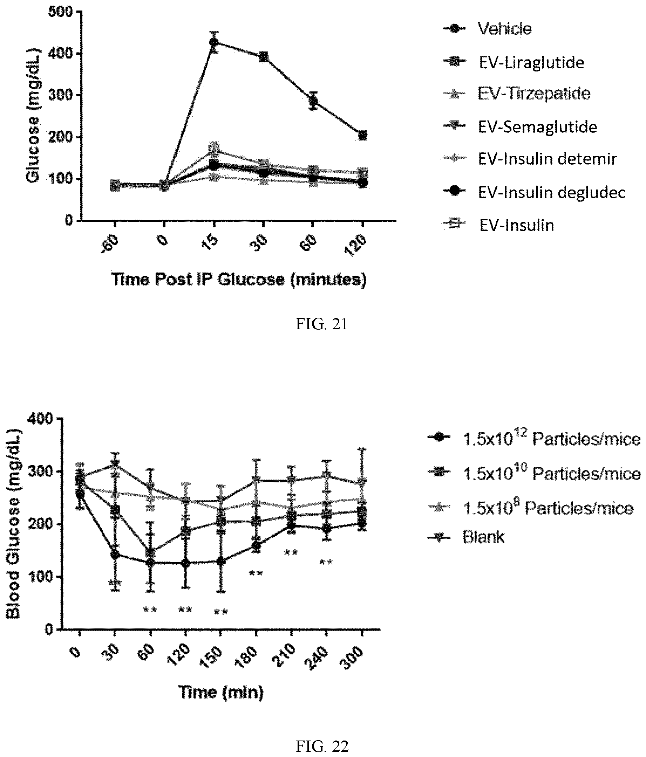

The results are shown in FIG. 21 and Table 15.

**Table 15**

| Time (min) | -60 | 0 | 15 | 30 | 60 | 120 |
|---|---|---|---|---|---|---|
| Vehicle | 80 | 81 | 431 | 398 | 298 | 199 |
| | 83 | 85 | 452 | 382 | 301 | 203 |
| | 87 | 85 | 403 | 401 | 265 | 217 |
| Exosome-liraglutide | 87 | 88 | 135 | 121 | 110 | 100 |
| | 82 | 86 | 146 | 135 | 109 | 102 |
| | 85 | 81 | 132 | 129 | 101 | 90 |
| Exosome-tirzepatide | 91 | 93 | 101 | 99 | 95 | 93 |
| | 87 | 81 | 110 | 97 | 94 | 90 |
| | 89 | 84 | 109 | 98 | 92 | 88 |
| Exosome-semaglutide | 92 | 89 | 129 | 112 | 109 | 98 |
| | 91 | 93 | 137 | 130 | 110 | 95 |
| | 86 | 83 | 140 | 129 | 103 | 100 |
| Exosome-insulin detemir | 86 | 88 | 139 | 120 | 106 | 90 |
| | 83 | 89 | 125 | 111 | 103 | 92 |
| | 82 | 80 | 131 | 109 | 101 | 95 |
| Exosome-insulin degludec | 88 | 81 | 135 | 121 | 107 | 95 |
| | 89 | 84 | 126 | 114 | 102 | 93 |
| | 81 | 89 | 139 | 117 | 109 | 90 |
| Exosome-insulin | 83 | 89 | 155 | 141 | 120 | 116 |
| | 81 | 88 | 189 | 139 | 126 | 120 |
| | 89 | 85 | 167 | 129 | 118 | 111 |

### Result analysis

The use of milk exosomes can achieve loading of multiple T2DM treatment drugs. The drug-loaded exosomes can achieve significant efficacy within 15 min after sublingual administration. And it can maintain the stable blood glucose and improve the glucose tolerance of mice.

### Example 10 In vivo efficacy (random blood glucose level) of sublingual tablets containing liraglutide-loaded milk exosome

By ultrasonication method, liraglutide (not limited to liraglutide, but also including insulin degludec, insulin detemir, semaglutide and tirzepatide) was loaded into milk exosomes and purified to obtain drug-loaded exosomes, which were prepared into sublingual tablets according to the optimal preparation formula in Example 8. Here, the results of milk exosomes loaded with liraglutide are illustrated as an example.

Diabetic mice were administered sublingually with different dosages of milk exosomes loaded with liraglutide, and random blood glucose levels of the mice were detected.

The results are shown in FIG. 22 and Table 16.

**Table 16**

| min | Blank (Unit: mg/dL of glucose) | | | | | | | | | |
|---|---|---|---|---|---|---|---|---|---|---|
| 0 | 277 | 245 | 288 | 266 | 329 | 312 | 289 | 287 | 290 | 321 |
| 30 | 302 | 282 | 340 | 299 | 340 | 340 | 323 | 319 | 307 | 285 |
| 60 | 215 | 234 | 265 | 221 | 298 | 284 | 290 | 290 | 279 | 320 |
| 120 | 194 | 194 | 238 | 213 | 259 | 251 | 283 | 283 | 261 | 266 |
| 150 | 210 | 219 | 253 | 220 | 223 | 225 | 275 | 274 | 281 | 272 |
| 180 | 225 | 211 | 327 | 340 | 298 | 291 | 284 | 284 | 273 | 297 |
| 210 | 235 | 267 | 262 | 273 | 320 | 322 | 296 | 291 | 286 | 279 |
| 240 | 306 | 259 | 243 | 249 | 299 | 316 | 314 | 316 | 307 | 309 |
| 300 | 169 | 150 | 296 | 255 | 320 | 303 | 340 | 333 | 320 | 286 |

| min | 1.5×10⁸ particles per mouse (Unit: mg/dL of glucose) | | | | | | | | | |
|---|---|---|---|---|---|---|---|---|---|---|
| 0 | 212 | 213 | 251 | 252 | 277 | 277 | 281 | 284 | 332 | 330 |
| 30 | 303 | 340 | 238 | 269 | 209 | 192 | 239 | 233 | 302 | 282 |
| 60 | 245 | 263 | 282 | 252 | 232 | 217 | 250 | 224 | 290 | 279 |
| 120 | 208 | 229 | 259 | 257 | 210 | 225 | 251 | 307 | 259 | 274 |
| 150 | 167 | 160 | 276 | 276 | 215 | 198 | 236 | 225 | 261 | 270 |
| 180 | 190 | 220 | 291 | 289 | 225 | 258 | 183 | 233 | 269 | 269 |
| 210 | 214 | 192 | 287 | 301 | 212 | 201 | 182 | 174 | 274 | 284 |
| 240 | 246 | 204 | 269 | 300 | 276 | 249 | 166 | 155 | 279 | 290 |
| 300 | 239 | 235 | 279 | 286 | 212 | 273 | 193 | 197 | 291 | 286 |

| min | 1.5×10¹⁰ particles per mouse (Unit: mg/dL of glucose) | | | | | | | | | |
|---|---|---|---|---|---|---|---|---|---|---|
| 0 | 298 | 254 | 271 | 253 | 292 | 281 | 299 | 274 | 310 | 302 |
| 30 | 340 | 333 | 166 | 156 | 258 | 272 | 198 | 176 | 192 | 192 |
| 60 | 207 | 205 | 75 | 78 | 197 | 169 | 89 | 82 | 179 | 189 |
| 120 | 209 | 199 | 95 | 91 | 212 | 210 | 187 | 285 | 192 | 196 |
| 150 | 188 | 190 | 166 | 186 | 220 | 240 | 219 | 211 | 227 | 219 |
| 180 | 191 | 194 | 151 | 165 | 218 | 232 | 229 | 221 | 231 | 228 |
| 210 | 237 | 219 | 162 | 156 | 221 | 229 | 235 | 230 | 239 | 239 |
| 240 | 226 | 175 | 211 | 199 | 229 | 231 | 239 | 231 | 228 | 240 |
| 300 | 221 | 202 | 209 | 206 | 237 | 230 | 238 | 229 | 242 | 241 |

| min | 1.5×10¹² particles per mouse (Unit: mg/dL of glucose) | | | | | | | | | |
|---|---|---|---|---|---|---|---|---|---|---|
| 0 | 284 | 271 | 283 | 294 | 277 | 245 | 238 | 226 | 231 | 233 |
| 30 | 115 | 118 | 151 | 158 | 246 | 282 | 105 | 85 | 94 | 83 |
| 60 | 125 | 113 | 116 | 126 | 215 | 234 | 85 | 104 | 90 | 71 |
| 120 | 124 | 138 | 137 | 138 | 194 | 194 | 115 | 118 | 56 | 58 |
| 150 | 133 | 130 | 149 | 121 | 210 | 219 | 129 | 135 | 39 | 44 |
| 180 | 151 | 161 | 181 | 143 | 155 | 151 | 176 | 169 | 162 | 156 |
| 210 | 216 | 218 | 194 | 197 | 195 | 207 | 194 | 194 | 176 | 197 |
| 240 | 222 | 226 | 174 | 154 | 176 | 199 | 194 | 196 | 188 | 197 |
| 300 | 229 | 207 | 209 | 204 | 179 | 210 | 195 | 193 | 202 | 204 |

Conclusion: From the results of random blood glucose levels, it could be seen that the exosomes loaded with liraglutide can significantly lower blood glucose levels in diabetic mice.

### Example 11 In vivo efficacy (fasting blood glucose level) of sublingual tablets containing liraglutide-loaded milk exosome

By electroporation, liraglutide (not limited to liraglutide, but also including insulin degludec, insulin detemir, semaglutide and tirzepatide) was loaded into milk exosomes and purified to obtain drug-loaded exosomes, which were prepared into sublingual tablets. Here, the results of milk exosomes loaded with liraglutide are illustrated as an example.

Diabetic mice were fasted for 6 h, and then orally administered with different concentrations of milk exosomes loaded with liraglutide. The fasting blood glucose of the mice was detected.

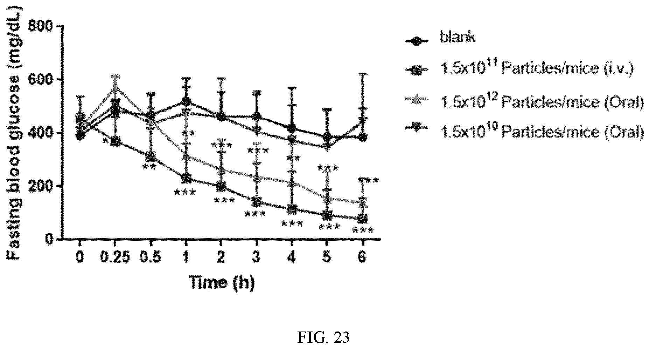

The results are shown in FIG. 23 and Table 17.

**Table 17**

| h | Blank (Unit: mg/dL of glucose) | | | | | | | | | |
|---|---|---|---|---|---|---|---|---|---|---|
| 0 | 448.2 | 390.6 | 342 | 284.4 | 433.8 | 435.6 | 462.6 | 441 | 342 | 351 |
| 0.25 | 457.2 | 439.2 | 423 | 473.4 | 468 | 522 | 525.6 | 570.6 | 466.2 | 487.8 |
| 0.5 | 460.8 | 392.4 | 399.6 | 361.8 | 459 | 451.8 | 599.4 | 585 | 473.4 | 495 |
| 1 | 612 | 612 | 374.4 | 421.2 | 493.2 | 486 | 612 | 612 | 486 | 495 |
| 2 | 612 | 612 | 365.4 | 342 | 480.6 | 473.4 | 428.4 | 403.2 | 451.8 | 466.2 |
| 3 | 430.2 | 376.2 | 448.2 | 478.8 | 612 | 586.8 | 493.2 | 451.8 | 388.8 | 361.8 |
| 4 | 504 | 444.6 | 376.2 | 381.6 | 522 | 568.8 | 363.6 | 401.4 | 322.2 | 313.2 |
| 5 | 338.4 | 329.4 | 493.2 | 459 | 531 | 495 | 376.2 | 340.2 | 259.2 | 246.6 |
| 6 | 612 | 545.4 | 329.4 | 309.6 | 388.8 | 322.2 | 379.8 | 354.6 | 327.6 | 298.8 |

| h | i.v. | | | | | | | | | |
|---|---|---|---|---|---|---|---|---|---|---|
| | 1.5×10¹⁰ particles per mouse (Unit: mg/dL of glucose) | | | | | | | | | |
| 0 | 594 | 525.6 | 331.2 | 342 | 448.2 | 466.2 | 518.4 | 502.2 | 421.2 | 392.4 |
| 0.25 | 450 | 424.8 | 241.2 | 282.6 | 385.2 | 424.8 | 480.6 | 466.2 | 277.2 | 277.2 |
| 0.5 | 361.8 | 419.4 | 259.2 | 194.4 | 338.4 | 334.8 | 428.4 | 446.4 | 160.2 | 190.8 |
| 1 | 196.2 | 235.8 | 111.6 | 109.8 | 230.4 | 264.6 | 450 | 457.2 | 127.8 | 117 |
| 2 | 122.4 | 108 | 82.8 | 82.8 | 253.8 | 257.4 | 417.6 | 415.8 | 142.2 | 126 |
| 3 | 64.8 | 73.8 | 66.6 | 55.8 | 88.2 | 90 | 419.4 | 417.6 | 72 | 86.4 |
| 4 | 55.8 | 37.8 | 50.4 | 45 | 45 | 48.6 | 399.6 | 370.8 | 52.2 | 52.2 |
| 5 | 50.4 | 45 | 50.4 | 55.8 | 36 | 36 | 279 | 271.8 | 54 | 55.8 |
| 6 | 54 | 43.2 | 59.4 | 57.6 | 34.2 | 32.4 | 212.4 | 230.4 | 41.4 | 36 |

| h | s.l. (sublingual) 1.5×10¹² particles per mouse (Unit: mg/dL of glucose) | | | | | | | | | |
|---|---|---|---|---|---|---|---|---|---|---|
| 0 | 502.2 | 428.4 | 352.8 | 351 | 414 | 451.8 | 435.6 | 471.6 | 351 | 423 |
| 0.25 | 516.6 | 599.4 | 558 | 504 | 532.8 | 612 | 612 | 576 | 612 | 612 |
| 0.5 | 511.2 | 487.8 | 379.8 | 437.4 | 496.8 | 453.6 | 457.2 | 466.2 | 428.4 | 365.4 |
| 1 | 612 | 594 | 180 | 216 | 329.4 | 300.6 | 257.4 | 244.8 | 228.6 | 223.2 |
| 2 | 457.2 | 462.6 | 151.2 | 135 | 210.6 | 271.8 | 262.8 | 210.6 | 257.4 | 226.8 |
| 3 | 417.6 | 498.6 | 120.6 | 115.2 | 241.2 | 235.8 | 196.2 | 153 | 185.4 | 199.8 |
| 4 | 462.6 | 493.2 | 95.4 | 88.2 | 196.2 | 136.8 | 192.6 | 169.2 | 163.8 | 169.2 |
| 5 | 336.6 | 347.4 | 73.8 | 66.6 | 118.8 | 108 | 95.4 | 106.2 | 154.8 | 160.2 |
| 6 | 286.2 | 316.8 | 77.4 | 64.8 | 70.2 | 73.8 | 100.8 | 90 | 169.2 | 156.6 |

| h | s.l. (sublingual) | | | | | | | | | |
|---|---|---|---|---|---|---|---|---|---|---|
| | 1.5×10¹⁰ particles per mouse (Unit: mg/dL of glucose) | | | | | | | | | |
| 0 | 462.6 | 428.4 | 469.8 | 428.4 | 415.8 | 369 | 426.6 | 482.4 | 324 | 273.6 |
| 0.25 | 612 | 612 | 450 | 451.8 | 612 | 576 | 540 | 532.8 | 318.6 | 356.4 |
| 0.5 | 318.6 | 284.4 | 417.6 | 426.6 | 612 | 612 | 500.4 | 477 | 315 | 396 |
| 1 | 552.6 | 592.2 | 379.8 | 372.6 | 426.6 | 469.8 | 612 | 581.4 | 388.8 | 385.2 |
| 2 | 570.6 | 581.4 | 354.6 | 336.6 | 612 | 590.4 | 552.6 | 500.4 | 266.4 | 250.2 |
| 3 | 487.8 | 415.8 | 475.2 | 480.6 | 592.2 | 554.4 | 367.2 | 383.4 | 158.4 | 145.8 |
| 4 | 577.8 | 583.2 | 221.4 | 266.4 | 612 | 612 | 273.6 | 288 | 154.8 | 149.4 |
| 5 | 455.4 | 432 | 358.2 | 295.2 | 532.8 | 538.2 | 304.2 | 279 | 135 | 133.2 |
| 6 | 594 | 612 | 612 | 612 | 349.2 | 410.4 | 468 | 482.4 | 153 | 151.2 |

Conclusion: From the results of the fasting blood glucose, it could be seen that the exosomes loaded with liraglutide can significantly reduce blood glucose in diabetic mice.

The use of a milk exosome in the preparation of drug-loaded complex and subsequent production of sublingual tablets provided by the present disclosure is described in detail above. The principle and implementation of the present disclosure are illustrated by using specific embodiments herein. The above descriptions of the embodiments are only used to facilitate understanding of the method and the core idea of the present disclosure. It should be noted that, several improvements and modifications may be made by those skilled in the art to the present disclosure without departing from the principle of the present disclosure, and these improvements and modifications also fall within the protection scope of the claims of the present disclosure.

## Claims

1. A method of drug loading, wherein a milk exosome is used as a drug carrier.

2. A method in preparation of sublingual tablets, wherein drug-loaded milk exosome is mixed with excipients to prepare sublingual tablets.

3. The method according to claim 1 or 2, wherein the drug includes liraglutide.

4. The method according to claim 1 or 2, wherein the drug includes a protein drug, preferably insulin degludec, insulin detemir, semaglutide or tirzepatide.

5. The method according to any one of claims 1 to 4, wherein ultrasonication, electroporation or co-incubation approach is used for loading of the drug into milk exosomes.

6. The method according to claim 5, wherein the ultrasonication is carried out with an input power level between 450W and 600W and a sonication time between 20min and 1h.

7. The method according to claim 5, wherein the electroporation is carried out with a voltage of 400V-500V and a capacitance of 300 µF-400 µF.

8. The method according to claim 5, wherein the co-incubation is carried out with the temperature of 42°C -60 °C and the time of 0. 5h-4h.

9. The method according to any one of claims 1 to 8, wherein the milk exosome is prepared by:
step 1, pre-treatment, wherein the pre-treatment comprises fat removal and/or protein removal;
step 2, preliminary purification and concentration, wherein the preliminarily purification and concentration is performed by tangential flow ultrafiltration;
step 3, downstream purification, wherein the downstream purification are performed by a method selected from the group consisting of CIMmultus QA column purification, Capto core 700 column purification, S400 size exclusion chromatography purification, and a combination thereof.

10. A sublingual tablet, wherein the sublingual tablet includes drug-loaded milk exosomes.

11. The sublingual tablet according to claim 10, wherein the sublingual tablet includes milk exosomes loaded with Liraglutide.

12. The sublingual tablet according to claim 11, wherein the sublingual tablet includes cross-linked sodium carboxymethyl cellulose, mannitol, purified water and magnesium stearate.

13. The sublingual tablet according to claim 11, wherein the sublingual tablet includes sodium carboxymethyl cellulose, mannitol, microcrystalline cellulose and magnesium stearate.

14. The sublingual tablet according to claim 10, wherein the sublingual tablet includes milk exosomes loaded with insulin degludec, insulin detemir, semaglutide or tirzepatide.

15. The sublingual tablet according to any one of claims 10 to 14, wherein the sublingual tablet is prescribed for the treatment of type 2 diabetes mellitus.
